# EUROPEAN PATENT SPECIFICATION

(11) **EP 1 283 709 B1**
(45) Date of publication and mention of the grant of the patent: **27.07.2005**
(21) Application number: 01933082.8
(22) Date of filing: 04.05.2001
(51) Int. Cl.: A61K 31/19, A61K 31/16, C07C 259/06

(54) **PEPTIDE DEFORMYLASE INHIBITORS**
INHIBITOREN VON PEPTID DEFORMYLASE
INHIBITEURS DE LA PEPTIDE DEFORMYLASE

(30) Priority: 05.05.2000 US 202288 P; 04.10.2000 US 237518 P
(43) Date of publication of application: 19.02.2003
(73) Proprietor: SmithKline Beecham Corporation, Philadelphia, PA 19101 (US)
(72) Inventor: AUBART, Kelly, M., King of Prussia, PA 19406 (US); CHRISTENSEN, Siegfried, B., IV, King of Prussia, PA 19406 (US); BRIAND, Jacques, King of Prussia, PA 19406 (US); CUMMINGS, Maxwell, David, Strafford, PA 19087 (US)
(74) Representative: Rowden, Janette Yvonne
(86) International application number: PCT/US2001/014578
(87) International publication number: WO 2001/085160

(56) References cited:
- EP-A- 0 196 184
- WO-A-99/06361
- WO-A-99/39704
- WO-A1-87/04152
- US-A- 3 282 986
- US-A- 4 988 733

## Description

The present invention relates to the use of novel anti-bacterial compounds, and pharmaceutical compositions containing these compounds as peptide deformylase inhibitors.

### BACKGROUND OF THE INVENTION

Bacterial initiator methionyl tRNA is modified by methionyl tRNA formyltransferase (FMT) to produce formyl-methionyl tRNA. The formyl methionine (f-met) is then incorporated at the N-termini of newly synthesized polypeptides. Polypeptide deformylase (PDF or Def) then deformylates primary translation products to produce N-methionyl polypeptides. Most intracelluar proteins are further processed by methionine amino peptidase (MAP) to yield the mature peptide and free methionine, which is recycled. PDF and MAP are both essential for bacterial growth, and PDF is required for MAP activity. This series of reactions is referred to as the methionine cycle (Figure 1) 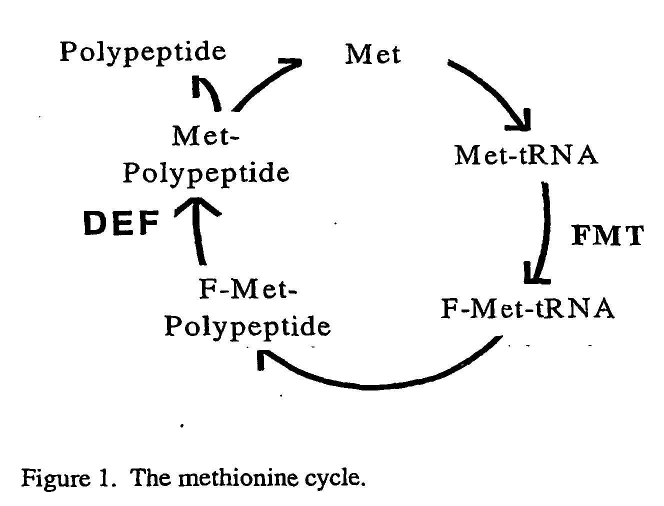 To date, polypeptide deformylase homologous genes have been found in bacteria, in chloroplast-containing plants, in mice and in humans. The plant proteins are nuclear encoded but appear to carry a chloroplast localisation signal. This is consistent with the observation that chloroplast RNA and protein synthesis processes are highly similar to those of eubacteria. No information on protein expression of mammalian PDF gene homologs or functional role for such proteins has been demonstrated to date (Meinnel T. 2000, Parasitology Today, 16(4), 165-168).

Polypeptide deformylase is found in all eubacteria for which high coverage genomic sequence information is available. Sequence diversity among PDF homologs is high, with as little as 20% identity between distantly related sequences. However, conservation around the active site is very high, with several completely conserved residues, including one cyteine and two histidines which are required to coordinate the active site metal (Meinnel, T. et al, 1997, Journal of Molecular Biology, 267, 749-761).

PDF is recognised to be an attractive anti-bacterial target, as this enzyme has been demonstrated to be essential for bacterial growth in vitro (Mazel, D. et al, EMBO J. 13 (4), 914-923, 1994), is not involved in eukaryotic protein synthesis (Rajagopalan et al, J. Am. Chem. Soc. 119, 12418-12419, 1997), and is universally conserved in prokaryotes (Kozak, M. Microbiol. Rev. 47, 1-45, 1983). Therefore PDF inhibitors can potentially serve as broad spectrum anti-bacterial agents.

WO99/06361A1 discloses reverse hydroxamate-containing compounds which inhibit matrix metalloproteinases, useful in the treatment of cancer.

WO99/39704A1 discloses N-formyl hydroxylamine derivatives as antibacterial agents.

### SUMMARY OF THE INVENTION

The present invention involves certain novel anti-bacterial compounds and their use as PDF inhibitors.

The present invention further provides the use of a compound of Formula (I) as indicated hereinbelow in the preparation of a medicament for use in the treatment of bacterial infections.

### DETAILED DESCRIPTION OF THE INVENTION

The compounds useful in the present invention are selected from Formula (I) hereinbelow: 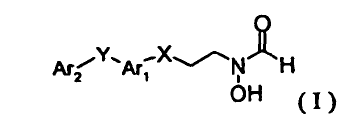 wherein,
X=O;
Ar1 is optionally substituted phenyl wherein the substituent is, independently, one, two, or three substituents selected from the group consisting of un-substituted alkyl or cycloalkyl having one to nine carbons, halo, alkoxy having one to nine carbons, hydroxy, hydroxyalkyl having one to nine carbons, alkoxyalkyl, wherein the alkyl and alkylene groups are independently of one to nine carbons;
Y is selected from the group consisting of a covalent bond, oxygen, alkylene of two to four carbon atoms, alkenylene of two carbon atoms, alkynylene of two carbon atoms, S(O)p, C(O), wherein p is 0-2;
Ar2 is an aryl group selected from the group consisting of phenyl, pyridyl, pyrazinyl, pyridazinyl, furyl, thienyl, isoxazolyl, oxazolyl, thiazolyl, and isothiazolyl; such that Ar2 may be optionally substituted with one, two, or three substituents selected from the group consisting of un-substituted alkyl or cycloalkyl having one to nine carbons, halo, alkoxy of one to nine carbons, hydroxy, hydroxyalkyl of one to nine carbons, alkoxyalkyl, wherein the alkyl and alkylene groups are independently of one to nine carbons; and cyano, provided that the compound according to formula (I) is other than N-[2-[(4'-cyano-[1,1'-biphenyl]-4-yl)oxylethyl]-N-hydroxyformamide.

As used herein, "alkyl" refers to an optionally substituted hydrocarbon group joined together by single carbon-carbon bonds. The alkyl hydrocarbon group may be linear, branched or cyclic, saturated or unsaturated. Preferably, the group is linear. Preferably, the group is saturated. Preferred alkyl moieties are C₁₋₄ alkyl, most preferably methyl.

As used herein, "aryl" refers to an optionally substituted aromatic group with at least one ring having a conjugated pi-electron system, containing up to two conjugated or fused ring systems. "Aryl" includes carbocyclic aryl, heterocyclic aryl and biaryl groups, all of which may be optionally substituted. Preferred aryl moieties are phenyl or naphthyl, unsubstituted, monosubstituted, disubstituted or trisubstituted.

Preferred compounds useful in the present invention are selected from the group consisting of:
N-Formyl-N-hydroxy-2-(*m*-biphenoxy)ethylamine;
N-Formyl-N-hydroxy-2-[(4-phenoxy)phenoxy]ethylamine; and
N-Formyl-N-hydroxy-2-(3-benzoylphenoxy)ethylamine.

Also included in the present invention are pharmaceutically acceptable salts and complexes. Preferred are the hydrochloride, hydrobromide and trifluoroacetate salts. The compounds of the present invention may contain one or more asymmetric carbon atoms and may exist in racemic and optically active forms. All of these compounds and diastereomers are contemplated to be within the scope of the present invention.

The compounds and processes of the present invention will be better understood in connection with the following synthetic schemes, which are merely illustrative of the methods by which the compounds of the invention may be prepared.and are not intended to limit the scope of the invention as defined in the appended claims. Representative procedures are outlined in the following Schemes 1 and 2.

Abbreviations which have been used in the descriptions of the schemes and the examples that follow are: THF for tetrahydrofuran and DMF for N,N-dimethylformamide.

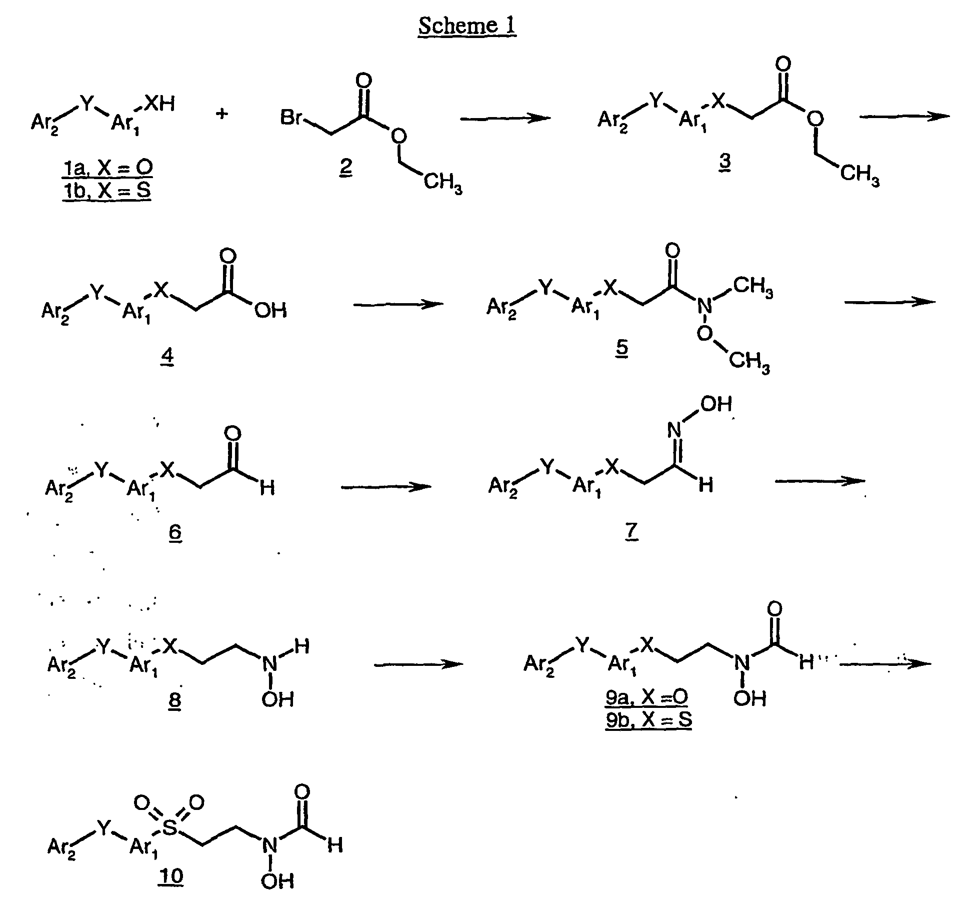

As shown in Scheme 1, alkylation of phenol 1a-Scheme 1 or aryl thiol 1b-Scheme 1 with ethyl bromoacetate (2-Scheme-1) can be accomplished in the presence of base, preferably potassium carbonate, in a polar solvent, preferably DMF, to provide 3-Scheme 2, which can be subsequently hydrolyzed to 4-Scheme 1 by treatment with aqueous base, preferably lithium hydroxide in a solvent mixture, preferably water and dioxane. Amide 5-Scheme 1 can be prepared by coupling N,O-dimethylhydroxylamine hydrochloride to 4-Scheme 1 with a coupling agent, preferably bis(2-oxo-3-oxazolidinyl)phosphinic chloride (BOP-Cl). Treatment of 5-Scheme 1 with a reducing agent, preferably excess lithium aluminum hydride or diisobutyl aluminum hydride, in an inert solvent, preferably THF, provides aldehyde 6-Scheme 1. This aldehyde can be converted to the corresponding oxime 7-Scheme 1 by treatment with hydroxylamine hydrochloride in a hydroxylic solvent, preferably ethanol, with a catalytic amound of base, preferably pyridine. Treatment of 7-Scheme 1 with a reducing agent, preferably borane-pyridine complex, in a hydroxylic solvent, preferably ethanol, and adding excess aqueous hydrochloride acid provides the correponding hydroxylamine 8-Scheme 1, which can be treated with a formylating agent, preferably formicacetyl anhydride, in solvents such a THF or dichloromethane, to provide hydroxamic acid 9-Scheme 1. The comppounds wherein X = S can be converted to the sulfones 10-Scheme 1 via oxidation with an appropriate oxidant, such as m-chloroperbenzoic acid or oxone.

Depending on the nature of substutuent groups of Ar₁ and Ar₂, protection and subsequent deprotection of other reactive groups may be required to successfully complete the described synthetic sequences. Commonly used protecting groups are disclosed in Greene, "Protective Groups in Organic Synthesis," (John Wiley & Sons, New York (1981)).

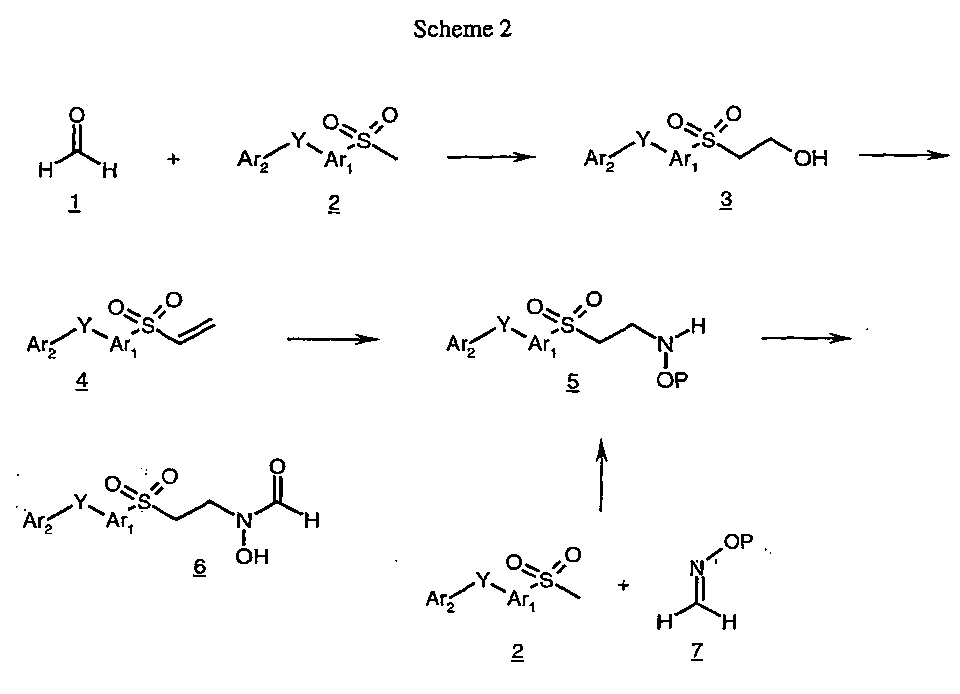

Scheme 2 illustrates an alternate synthesis of the sulfones 6-Scheme 2. Deprotonation of the sulfone 2-Scheme 2 with a base, such as LDA, followed by addition to a formaldehyde 1-Scheme 2 gives alcohol 3-Scheme 2, which can be dehydrated either by reaction with acid, such a toluene sulfonic acid, or by a stepwide 2-step procedure (first convering the alcohol into a leaving group, such as mesylate via treatment with mesyl chloride and triethylamine, then eliminating with a base, preferably 1,8-diazabicyclo[5.4.0]undec-7-ene). Reaction of the olefin 4-Scheme 2 with an O-protected hydroxylamine, preferably O-benzyl, gives the adduct 5-Scheme 2. The sulfone 5-Scheme 2 can also be prepared directly via the deprotonation of sulfone 2-Scheme 2 with a base, such as n-BuLi, and subsequent addition, preferably in the presence of boron trifluoride etherate, to O-protected oxime 7-Scheme 2. Formylation of 5-Scheme 2 as previously described in Scheme 1, followed by removal of the protecting group, preferably under hydrogenation conditions for the compounds wherein P is benzyl, affords the sulfone 6-Scheme 2.

Scheme 3 illustrates an alternative novel synthesis of the claimed compounds. Oxime 2-Scheme-3 can be prepared by treatment of aldehyde 1-Scheme-3 with hydroxylamine hydrochloride in a solvent such as pyridine. Reduction of the oxime using sodium cyanoborohydride under acidic conditions provides hydroxylamine 3-Scheme-3, and formylation using the mixed anhydride prepared from formic acid and acetic anhydride provides N-formyl-N-hydroxylamine 4-Scheme-3. The N-formyl-N-hydroxylamine 4-Scheme-3 is then loaded onto 2-chloro-trityl resin using a base such as triethylamine in a solvent such as dichloromethane. Resin-bound 5-Scheme-3 is then deprotected using tetrabutylammonium fluoride in tetrahydrofuran, providing 6-Scheme-3. Treatement of the free hydroxyl with aromatic alcohols under Mitsunobu conditions, followed by cleavage from the resin (5% TFA/dichloromethane) provides aryl N-formyl-N-hydroxylamines 8-Scheme-3.

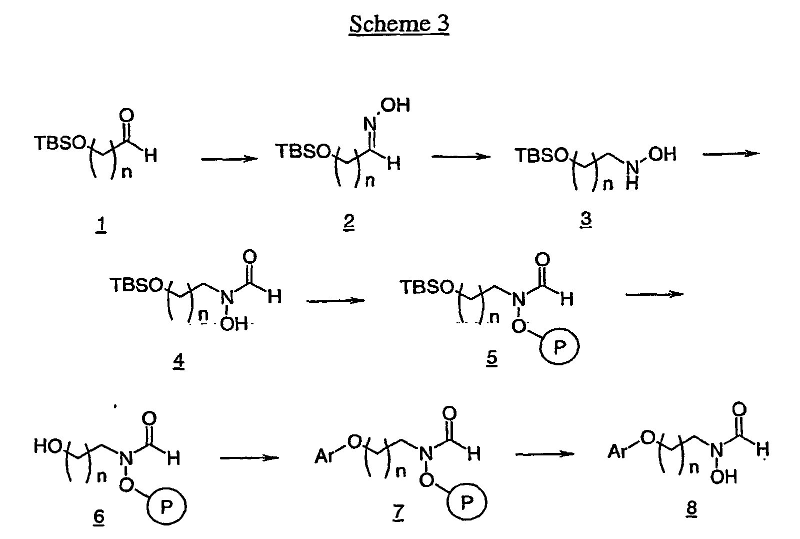

Alternatively, the compounds of formula I may be prepared as described in Scheme 4. Oxime 2-Scheme-4 can be prepared by treatment of aldehyde 1-Scheme-4 with O-benzylhydroxylamine hydrochloride in a solvent such as pyridine. Reduction of the oxime using sodium cyanoborohydride under acidic conditions provides O-benzyloxyamine 3-Scheme-4, and formylation using the mixed anhydride prepared from formic acid and acetic anhydride provides N-formyl-N-(O-benzyloxy)amine 4-Scheme-4. The N-formyl-N-(O-benzyloxy)amine 4-Scheme-4 is then deprotected using tetrabutylammonium fluoride in tetrahydrofuran, providing 5-Scheme-4. Treatement of the free hydroxyl with aromatic alcohols under Mitsunobu conditions, followed by catalytic hydrogenation using Pd/C provides aryl N-formyl-N-hydroxylamines 7-Scheme-4.

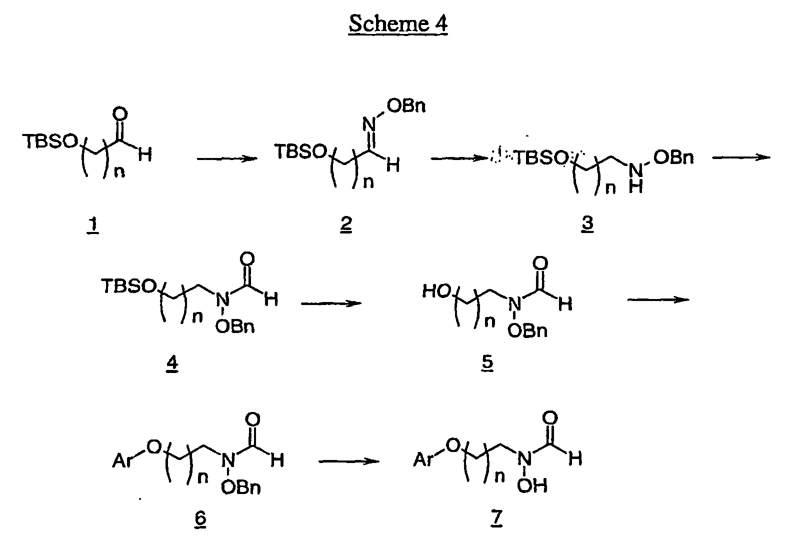

The foregoing may be better understood by reference to the following examples which illustrate the methods by which the compounds of the invention may be prepared and are not intended to limit the scope of the invention as defined in the appended claims.

### Example 1

N-Formyl-N-hydroxy-2-(*m*-biphenoxy)ethylamine, 1a. A solution of *t*-butyldimethylsilyloxyacetaldehyde (500 mg) in pyridine (5 mL) was treated with O-benzylhydroxylamine hydrochloride (551 mg) and stirred 90 min. The reaction solution was diluted with dichloromethane and extracted with 1M HCl. The organics were dried and concentrated to provide the oxime (784 mg) as a 1:1 mixture of *cis* and *trans* isomers as a colorless oil.
1c. To a solution of the above oxime (784 mg) in methanol (14 mL) at 0 °C was added 2 mg of methyl orange. With stirring, sodium cyanoborohydride (230 mg) was added slowly while simultaenously adding a solution of 6M HCl/methanol (1/1) dropwise as necessary to maintain the pink color of the methyl orange indicator. After stirring at 0 °C for 1 h, the reaction was brought to pH 9 with 6M NaOH, and the reaction was extracted with dichloromethane. The organics were dried and concentrated to provide the reduced amine (780 mg) as a colorless oil.
1d. Treatment of the O-benzyloxylamine (460 mg) with triethylamine (280 mg) and the mixed anhydride prepared from formic acid (127 mg) and acetic anhydride (283 mg) in dichloromethane (8 mL), followed by an extractive workup using 1M HCl provided the N-formyl-N-benzyloxyamine (490 mg).
1e. To a solution of the above N-formyl-N-benzyloxyamine (490 mg) in THF (7 mL) was added TBAF (1.67 mL of a 1M THF solution). After stirring for 45 min, the reaction was partitioned between ethyl acetate and water, and the layers were separated. The organics were dried and concentrated to provide the alcohol (300 mg).
1f. To a solution of the above alcohol (70 mg) and *m*-phenylphenol (61 mg) in THF (2 mL) was added diisopropylazodicarboxylate (80 mg), followed by triphenylphosphine (94 mg). After stirring overnight, the reaction mixture was eluted through a silica gel column (eluting with 25% ethyl acetate/hexanes): Concentration of the appropriate collected fractions provided the aryl ether (115 mg).
1g. A suspension of the above aryl ether (115 mg) and Pd/C (20 mg) in methanol (6 mL) was stirred under an atmosphere of hydrogen for 90 min. Filtration of the reaction mixture through a pad of Celite and concentration of the filtrate provided the crude N-formyl-N-hydroxylamine. Purification by reverse-phase HPLC provided N-Formyl-N-hydroxy-2-(*m*-biphenoxy)ethylamine, as a white solid.

### General procedure for synthesis of N-formyl-N-hydroxylamine ethers

1a. A solution of t-butyldimethylsilyloxy-acetaldehyde in pyridine was treated with hydroxylamine hydrochloride and stirred overnight. The reaction solution was diluted with dichloromethane and extracted with 1M HCl. The organics were dried and concentrated to provide the oxime as a 1:1 mixture of cis and trans isomers as a colorless oil.
1b. To a solution of the above oxime in methanol at 0 °C was added 2 mg of methyl orange. With stirring, sodium cyanoborohydride was added slowly while simultaenously adding a solution of 6M HCl/methanol (1/1) dropwise as necessary to maintain the pink color of the methyl orange indicator. After stirring at 0 °C for 1 h, the reaction was brought to pH 9 with.6M NaOH, and the reaction was extracted with dichloromethane. The organics were dried.and concentrated to provide the hydroxylamine as a colorless oil.
1c. Treatment of the hydroxylamine with 1 equivalent of the mixed anhydride prepared from formic acid and acetic anhydride (1:1) and 1 equivalent of triethylamine in dichloromethane, followed by an extractive workup using dichloromethane and 1M HCl, provides the N-formyl-N-hydroxylamine as shown in 4-Scheme-3. Loading of the N-formyl-N-hydroxylamine onto resin is accomplished by shaking a solution of 2-chlorotrityl resin, the N-formyl-N-hydroxylamine, and triethylamine in dichloromethane overnight. The resin is then washed with dichloromethane, tetrahydrofuran, and again with dichloromethane. Treatment of the loaded resin with TBAF in THF and shaking for 3 hours, followed by washing with tetrahydrofuran, dichloromethane, methanol, and again with dichloromethane, provides the free alcohol on the resin. Treatment of the alcohol with the appropriate aromatic alcohol under Mitsunobu conditions (DLAD, PPh3, THF) overnight, followed by washing with tetrahydrofuran (3 times), dichloromethane, DMF, tetrahydrofuran, and dichloromethane, provides the aromatic ethers as in 7-Scheme 3. Cleavage of the products from support is accomplished by treating the resin with a solution of 5% TFA in methanol for 15 min, followed by washing with dichloromethane then methanol. The filtrate is then concentrated and purified by high-throughput reverse-phase HPLC to provide the ethers such as 8-Scheme-3.

According to this procedure, the following compounds were prepared:
N-Formyl-N-hydroxy-2-[(4-phenoxy)phenoxy]ethylamine, white solid.
N-Formyl-N-hydroxy-2-(3-benzoylphenoxy)ethylamine, white solid.

With appropriate manipulation and protection of any chemical functionality, synthesis of the remaining compounds of Formula (I) is accomplished by methods analogous to those above and to those described in the Experimental section.

In order to use a compound of the Formula (I) or a pharmaceutically acceptable salt thereof for the treatment of humans and other mammals it is normally formulated in accordance with standard pharmaceutical practice as a pharmaceutical composition.

The present compounds are useful for the treatment of bacterial infections including but not limited to respiratory tract infections and/or Gram positive infections.

Compounds of Formula (I) and their pharmaceutically acceptable salts may be administered in a standard manner for antibiotics, for example orally, parenterally, sub-lingually, dermally, transdermally, rectally, via inhalation or via buccal administration.

Compositions of Formula (I) and their pharmaceutically acceptable salts which are active when given orally can be formulated as syrups, tablets, capsules, creams and lozenges. A syrup formulation will generally consist of a suspension or solution of the compound or salt in a liquid carrier for example, ethanol, peanut oil, olive oil, glycerine or water with a flavoring or coloring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils, and are incorporated in a soft gelatin capsule shell.

Typical parenteral compositions consist of a solution or suspension of a compound or salt in a sterile aqueous or non-aqueous carrier optionally containing a parenterally acceptable oil, for example polyethylene glycol, polyvinylpyrrolidone, lecithin, arachis oil or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of Formula (I) or a pharmaceutically acceptable salt thereof which is active when administered in this way, with a binding and/or lubricating agent, for example polymeric glycols, gelatins, cocoa-butter or other low melting vegetable waxes or fats or their synthetic analogs.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

Each dosage unit for oral administration contains suitably from 0.1 mg to 500 mg/Kg, and preferably from 1 mg to 100 mg/Kg, and each dosage unit for parenteral administration contains suitably from 0.1 mg to 100 mg/Kg, of a compound of Formula(I) or a pharmaceutically acceptable salt thereof calculated as the free acid. Each dosage unit for intranasal administration contains suitably 1-400 mg and preferably 10 to 200 mg per person. A topical formulation contains suitably 0.01 to 5.0% of a compound of Formula (I).

The daily dosage regimen for oral administration is suitably about 0.01 mg/Kg to 40 mg/Kg, of a compound of Formula(I) or a pharmaceutically acceptable salt thereof calculated as the free acid. The daily dosage regimen for parenteral administration is suitably about 0.001 mg/Kg to 40 mg/Kg, of a compound of Formula (I) or a pharmaceutically acceptable salt thereof calculated as the free acid, the daily dosage regimen for intranasal administration and oral inhalation is suitably about 10 to about 500 mg/person. The active ingredient may be administered from 1 to 6 times a day, sufficient to exhibit the desired activity.

No unacceptable toxicological effects are expected when compounds of the present invention are administered in accordance with the present invention.

The biological activity of the compounds of Formula (I) are demonstrated by the following test:

### Biological Assay:

S. Aureus or E. Coli PDF activity is measured at 25°C, using a continuous enzyme-linked assay developed by Lazennec & Meinnel, (1997) "Formate dehydrogenase-coupled spectrophotometric assay of peptide deformylase" Anal. Biochem. 244, pp.180-182, with minor modifications. The reaction mixture is contained in 50 uL with 50 mM potassium phosphate buffer (pH7.6), 15 mM NAD, 0.25 U formate dehydrogenase. The substrate peptide, f-Met-Ala-Ser, is included at the K_{M} concentration. The reaction is triggered with the addition of 10 nM Def1 enzyme, and absorbance is monitored for 20 min at 340 nm.

### Antimicrobial Activity Assay

Whole-cell antimicrobial activity was determined by broth microdilution using the National Committee for Clinical Laboratory Standards (NCCLS) recommended procedure, Document M7-A4, "Methods for Dilution Susceptibility Tests for Bacteria that Grow Aerobically" (incorporated by reference herein). The compound was tested in serial two-fold dilutions ranging from 0.06 to 64 mcg/ml. A panel of 12 strains were evaluated in the assay. This panel consisted of the following laboratory strains: Staphylococcus aureus Oxford, Streptococcus pneumoniae R6, Streptococcus pyogenes CN10, Enterococcus faecalis I, Haemophilus influenzae Q1, Escherichia coli DC0, E. coli EES, E. coli 7623 (AcrAB+) E. coli 120 (AcrAB-) Klebsiella pneumoniae E70, Pseudomonas aeruginosa K799wt and Candida albicans GRI 681. The minimum inhibitory concentration (MIC) was determined as the lowest concentration of compound that inhibited visible growth. A mirror reader was used to assist in determining the MIC endpoint.

## Claims

1. Use of a compound of formula (I) 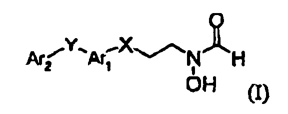 wherein,
X=O;
Ar1 is optionally substituted phenyl wherein the substituent is, independently, one, two, or three substituents selected from the group consisting of un-substituted alkyl or cycloalkyl having one to nine carbons, halo, alkoxy having one to nine carbons, hydroxy, hydroxyalkyl having one to nine carbons, alkoxyalkyl, wherein the alkyl and alkylene groups are independently of one to nine carbons;
Y is selected from the group consisting of a covalent bond, oxygen, alkylene of two to four carbon atoms, alkenylene of two carbon atoms, alkynylene of two carbon atoms, S(O)p, C(O), wherein p is 0-2;
Ar2 is an aryl group selected from the group consisting of phenyl, pyridyl, pyrazinyl, pyridazinyl, furyl, thienyl, isoxazolyl, oxazolyl, thiazolyl, and isothiazolyl; such that Ar2 may be optionally substituted with one, two, or three substituents selected from the group consisting of un-substituted alkyl or cycloalkyl having one to nine carbons, halo, alkoxy of one to nine carbons, hydroxy, hydroxyalkyl of one to nine carbons, alkoxyalkyl, wherein the alkyl and alkylene groups are independently of one to nine carbons; and cyano, or pharmaceutically acceptable salts or complexes thereof, in the preparation of a medicament for use in the treatment of bacterial infections.

2. A compound selected from the group consisting of:
N-Formyl-N-hydroxy-2-(*m*-biphenoxy)ethylamine;
N-Formyl-N-hydroxy-2-[(4-phenoxy)phenoxy]ethylamine; and
N-Formyl-N-hydroxy-2-(3-benzoylphenoxy)ethylamine, or pharmaceutically acceptable salts or complexes thereof.

3. A compound according to claim 2, or pharmaceutically acceptable salts or complexes thereof, for use as a PDF inhibitor.

4. A compound according to claim 2, or pharmaceutically acceptable salts or complexes thereof, for use in the treatment of bacterial infections.

5. Use of a compound according to claim 2, or pharmaceutically acceptable salts or complexes thereof, in the preparation of a medicament for use in the treatment of bacterial infections.

6. A compound according to claim 4 wherein the bacterial infection is respiratory tract infection (RTI) and/or the Gram + TPP.

7. A compound according to claim 6 wherein the Gram + TPP is staphylococci, streptococci and/or enterococci.

8. Use according to claim 5 wherein the bacterial infection is respiratory tract infection (RTI) and/or the Gram + TPP.

9. Use according to claim 8 wherein the Gram + TPP is staphylococci, streptococci and/or enterococci.

10. A pharmaceutical composition comprising at least one compound according to claim 2, or pharmaceutically acceptable salts or complexes thereof.

## Patentansprüche

1. Verwendung einer Verbindung der Formel (I) 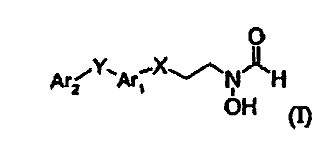 wobei
X=O;
Ar₁ gegebenenfalls substituiertes Phenyl ist, wobei der Substituent unabhängig ein, zwei oder drei Substituenten ist, ausgewählt aus einem unsubstituierten Alkyl oder Cycloalkyl mit 1 bis 9 Kohlenstoffatomen, Halogen, Alkoxy mit 1 bis 9 Kohlenstoffatomen, Hydroxy, Hydroxyalkyl mit 1 bis 9 Kohlenstoffatomen, Alkoxyalkyl, wobei die Alkyl- und Alkylenreste unabhängig 1 bis 9 Kohlenstoffatome aufweisen;
Y ausgewählt ist aus einer covalenten Bindung, Sauerstoff, Alkylen mit 2 bis 4 Kohlenstoffatomen, Alkenylen mit 2 Kohlenstoffatomen, Alkinylen mit zwei Kohlenstoffatomen, S(O)ₚ, C(O), wobei p gleich 0 bis 2 ist;
Ar₂ ein Arylrest ist, ausgewählt aus Phenyl, Pyridyl, Pyrazinyl, Pyridazinyl, Furyl, Thienyl, Isoxazolyl, Oxazolyl, Thiazolyl und Isothiazolyl, so dass Ar₂ gegebenenfalls mit einem, zwei oder drei Substituenten substituiert sein kann, ausgewählt aus einem unsubstituierten Alkyl oder Cycloalkyl mit 1 bis 9 Kohlenstoffatomen, Halogen, Alkoxy mit 1 bis 9 Kohlenstoffatomen, Hydroxy, Hydroxyalkyl mit 1 bis 9 Kohlenstoffatomen, Alkoxyalkyl, wobei die Alkyl- und Alkylenreste unabhängig 1 bis 9 Kohlenstoffatome aufweisen und Cyano, oder pharmazeutisch verträgliche Salze oder Komplexe davon zur Herstellung eines Medikament zur Verwendung in der Behandlung von bakteriellen Infektionen.

2. Verbindung, ausgewählt aus:
N-Formyl-N-hydroxy-2-(*m*-biphenoxy)ethylamin;
N-Formyl-N-hydroxy-2-[(4-phenoxy)phenoxy]ethylamin; und
N-Formyl-N-hydroxy-2-(3-benzoylphenoxy)ethylamin oder pharmazeutisch verträgliche Salze oder Komplexe davon.

3. Verbindung gemäß Anspruch 2 oder pharmazeutisch verträgliche Salze oder Komplexe davon, zur Verwendung als ein PDF-Hemmer.

4. Verbindung gemäß Anspruch 2 oder pharmazeutisch verträgliche Salze oder Komplexe davon, zur Verwendung in der Behandlung von bakteriellen Infektionen.

5. Verwendung einer Verbindung gemäß Anspruch 2 oder pharmazeutisch verträglicher Salze oder Komplexe davon, zur Herstellung eines Medikaments zur Verwendung in der Behandlung von bakteriellen Infektionen.

6. Verbindung gemäß Anspruch 4, wobei die bakterielle Infektion eine Atemwegsinfektion (RTI) und/oder Gram + TPP ist.

7. Verbindung gemäß Anspruch 6, wobei Gram + TPP Staphylokokken, Streptokokken und/oder Enterokokken sind.

8. Verwendung gemäß Anspruch 5, wobei die bakterielle Infektion eine Atemwegsinfektion (RTI) und/oder Gram + TPP ist.

9. Verwendung gemäß Anspruch 8, wobei Gram + TPP Staphylokokken, Streptokokken und/oder Enterokokken sind.

10. Arzneimittel, umfassend mindestens eine Verbindung gemäß Anspruch 2 oder pharmazeutisch verträgliche Salze oder Komplexe davon.

## Revendications

1. Utilisation d'un composé de formule (I) 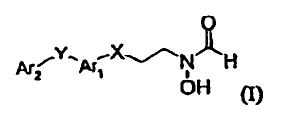 dans laquelle,
X représente un atome de O ;
Ar1 représente un groupe phényle facultativement substitué dans lequel le substituant consiste, indépendamment, en un, deux ou trois substituants choisis dans le groupe consistant en des substituants alkyle ou cycloalkyle non substitués ayant un à neuf atomes de carbone, halogéno, alkoxy ayant un à neuf atomes de carbone, hydroxy, hydroxyalkyle ayant un à neuf atomes de carbone, alkoxyalkyle, dans lesquels les groupes alkyle et alkylène possèdent indépendamment un à neuf atomes de carbone ;
Y est choisi dans le groupe consistant en une liaison covalente, un atome d'oxygène, un groupe alkylène ayant deux à quatre atomes de carbone, un groupe alcénylène ayant deux atomes de carbone, un groupe alcynylène ayant deux atomes de carbone, un groupe S(O)p et un groupe C(O), dans lesquels p a une valeur de 0 à 2 ;
Ar2 représente un groupe aryle choisi dans le groupe consistant en les groupes phényle, pyridyle, pyrazinyle, pyridazinyle, furyle, thiényle, isoxazolyle, oxazolyle, thiazolyle et isothiazolyle ; de telle sorte que Ar2 puisse être facultativement substitué avec un, deux ou trois substituants choisis dans le groupe consistant en des substituants alkyle ou cycloalkyle non substitués ayant un à neuf atomes de carbone, halogéno, alkoxy ayant un à neuf atomes de carbone, hydroxy, hydroxyalkyle ayant un à neuf atomes de carbone, alkoxyalkyle, dans lesquels les groupes alkyle et alkylène ont indépendamment un à neuf atomes de carbone ; et cyano, ou de ses sels ou complexes pharmaceutiquement acceptables, dans la préparation d'un médicament destiné à être utilisé dans le traitement d'infections bactériennes.

2. Composé choisi dans le groupe consistant en :
N-formyl-N-hydroxy-2-(*m*-biphénoxy)éthylamine ;
N-formyl-N-hydroxy-2-[(4-phénoxy)phénoxy]éthylamine ; et
N-formyl-N-hydroxy-2-(3-benzoylphénoxy)éthylamine, ou ses sels ou complexes pharmaceutiquement acceptables.

3. Composé suivant la revendication 2, ou ses sels ou complexes pharmaceutiquement acceptables, destinés à être utilisés comme inhibiteur de PDF.

4. Composé suivant la revendication 2, ou ses sels ou complexes pharmaceutiquement acceptables, destinés à être utilisés dans le traitement d'infections bactériennes.

5. Utilisation d'un composé suivant la revendication 2, ou de ses sels ou complexes pharmaceutiquement acceptables, dans la préparation d'un médicament destiné à être utilisé dans le traitement d'infections bactériennes.

6. Composé suivant la revendication 4, dans lequel l'infection bactérienne est une infection du tractus respiratoire (ITR) et/ou une infection TPP à microorganismes Gram-positifs.

7. Composé suivant la revendication 6, dans lequel les microorganismes TPP Gram-positifs sont des staphylocoques, des streptocoques et/ou des entérocoques.

8. Utilisation suivant la revendication 5, dans laquelle l'infection bactérienne est une infection du tractus respiratoire (ITR) et/ou une infection par des microorganismes TPP Gram-positifs.

9. Utilisation suivant la revendication 8, dans laquelle les microorganismes TPP Gram-positifs sont des staphylocoques, des streptocoques et/ou des entérocoques.

10. Composition pharmaceutique comprenant au moins un composé suivant la revendication 2, ou un de ses sels ou complexes pharmaceutiquement acceptables.
